# FASCICULE DE BREVET EUROPEEN

(11) **EP 2 134 264 B1**
(45) Date de publication et mention de la délivrance du brevet: **19.12.2012**
(21) Numéro de dépôt: 07858620.3
(22) Date de dépôt: 18.10.2007
(51) Int. Cl.: A61B 17/02, A61B 19/08

(54) **DISPOSITIF DE PROTECTION ACTIF DES BERGES D'INCISION**
VORRICHTUNG FÜR AKTIVEN SCHUTZ DER INZISIONSRÄNDER
DEVICE FOR ACTIVE PROTECTION OF THE MARGINS OF AN INCISION

(30) Priorité: 20.10.2006 FR 0609220
(43) Date de publication de la demande: 23.12.2009
(73) Titulaire: Touati, Gilles, 80000 Amiens (FR)
(72) Inventeur: Touati, Gilles, 80000 Amiens (FR)
(74) Mandataire: Roman, Alexis
(86) Numéro de dépôt international: PCT/FR2007/052197
(87) Numéro de publication internationale: WO 2008/047059

(56) Documents cités:
- DE-A1- 2 657 520
- GB-A- 2 221 620
- GB-A- 2 377 177
- US-A- 4 089 331

## Description

La présente invention a pour objet un dispositif de protection actif des berges d'incision.

Elle concerne le domaine technique des accessoires de chirurgie permettant de protéger les berges d'incision lors d'une intervention chirurgicale. Elle concerne plus particulièrement le domaine technique des champs de bordure chirurgicaux.

Lors des opérations de chirurgie, il est courant d'employer des champs de bordure chirurgicaux équipés de fenêtres d'incision au travers desquelles sont réalisés les actes chirurgicaux. Ces champs se placent sur la peau du patient et ont pour but d'isoler et de protéger la zone d'incision contre toute contamination. Ils forment une barrière efficace entre le corps du patient et l'atmosphère du bloc opératoire.

Les champs de bordure chirurgicaux habituellement employés sont fabriqués à partir d'un matériau stérile souple non tissé. Généralement, la face inférieure en contact avec la peau du patient est apte à absorber les sécrétions corporelles telles que les saignements tandis que la face supérieure est imperméable aux fluides. Il est courant d'employer des champs cutanés chirurgicaux iodés ou imprégnés d'une substance antimicrobienne, ayant un effet rémanent sur les microbes présents sur la peau du patient (essentiellement les staphylocoques cutanés). De tels champs sont par exemple décrits dans les documents WO 2005/110082 (3M), WO 00/71183 (3M), EP 0.812.893 (MEDICAL CONCEPTS DEVELOPMENT), EP 0.240.097 (SURGIKOS), EP 0.136.900 (SURGIKOS) ou encore FR 2.012.584 (T.J. SMITH et al). Ces champs peuvent être adhésifs de manière à parfaitement rester en place tout au long de l'intervention et border au plus près la zone d'incision.

Bien que couramment employés, ces champs de bordure chirurgicaux ne sont pas totalement efficaces dans la mesure où ils ne sont que cutanés et ne protègent pas les berges d'incision. En effet, dès que la peau est incisée, un saignement même minime de ces tissus et l'exposition prolongée à l'air fait que ces berges d'incision présentent un risque de voir se développer des infections, des abcès ou des hématomes tout à fait néfastes pour la santé du patient.

Pour tenter de résoudre ce problème technique, on connaît par le document WO 99/03416 (MEDICAL CREATIVE TECHNOLOGIES), l'utilisation d'un élément tubulaire souple rétractable que l'on positionne autour des berges d'incision. Cet élément tubulaire est toutefois difficile à mettre en place par le praticien, car sa manipulation est complexe. En outre, il est nécessaire de prévoir des éléments tubulaires de différents diamètres de façon à pouvoir s'adapter à la taille de l'incision réalisée, ce qui est peu pratique et coûteux.

Le document US 4.089.331 (THE KANDALL COMPANY) tente également d'apporter une solution en proposant d'utiliser une compresse stérile rapportée à l'extrémité d'un drap chirurgical, le long du bord de la fenêtre d'incision. Cette compresse est aménagée de manière à être dans une position de rangement avant l'incision - rangée dans une poche spécifique ou rabattue sur le dessus du drap - et dans une position venant recouvrir les berges d'incision après l'incision. La conception de ce dispositif apparaît être particulièrement complexe du fait qu'il faille prévoir une poche de rangement ou un dispositif permettant de maintenir la compresse en position rabattue. De plus, la longueur de la compresse ne peut être facilement ajustée de sorte que ladite compresse peut gêner l'intervention du praticien lorsqu'elle n'est pas adaptée à la profondeur de l'incision. Également, cette compresse n'est pas particulièrement efficace contre les infections ou contre la formation d'abcès ou d'hématomes.

Le document GB 2.221.620 (Johnson & Johnson) divulgue un pansement comprenant un substrat fibreux dont la surface est recouverte d'un alginate acceptable en pharmacologie. Ce type de pansement n'est toutefois pas spécifiquement adapté pour protéger les berges d'incision et éviter le développement des infections lors d'une intervention chirurgicale.

Le document GB 2.377.177 (ACORDIS) concerne un pansement comprenant une couche d'un matériau ultra-absorbant et une couche d'un matériau ayant des propriétés hémostatiques. Toutefois, ces différentes couches ne sont pas spécifiquement configurées pour que celle ayant des propriétés hémostatiques puisse venir recouvrir la partie supérieure et la partie inférieure d'une incision.

Face aux inconvénients de l'art antérieur et en particulier ceux du dispositif décrit dans US 4.089.331 (THE KANDALL COMPANY), le problème technique principal que vise à résoudre l'invention est de protéger efficacement, rapidement et durablement les berges d'incision sans que le praticien n'ait à effectuer des manipulations complexes.

La solution proposée par l'invention est un dispositif de protection actif des berges d'incision destiné à éviter le développement des infections, comportant un champ de bordure chirurgical dans lequel est aménagée une fenêtre d'incision totale ou partielle, et dans lequel :
- la face interne des bordures de la fenêtre d'incision est recouverte d'une première couche d'un matériau destiné à absorber les secrétions d'incision et d'une seconde couche imprégnée d'un produit hémostatique antiseptique ayant pour but d'éviter le développement des infections,
- la seconde couche vient couvrir partiellement la première couche, ladite première couche étant plus large que ladite seconde couche,
- la seconde couche est aménagée de manière à ce qu'elle puisse venir au contact des berges d'incision et recouvre la partie supérieure et la partie inférieure de l'incision lors de la mise en place d'un écarteur.

Ces caractéristiques techniques permettent au praticien de disposer directement les bordures de la fenêtre d'incision au niveau des berges d'incision pour protéger efficacement ces dernières.

Dans la compresse décrite dans le document US 4.089.331 (THE KANDALL COMPANY), la face interne des bordures de la fenêtre d'incision est recouverte d'une unique couche d'un matériau imperméable au passage des sécrétions corporelles et qui n'est donc pas adaptée pour absorber les sécrétions d'incision. De plus, les bordures ne comportent aucune autre couche spécifique imprégnée d'un produit hémostatique antiseptique. Également, le document US 4.089.331 (THE KANDALL COMPANY) ne précise pas que les bordures sont configurées pour venir au contact des berges d'incision et recouvrir la partie supérieure et la partie inférieure de l'incision lors de la mise en place d'un écarteur.

D'autres avantages et caractéristiques de l'invention apparaîtront mieux à la lecture de la description d'un mode de réalisation préférée qui va suivre, en référence aux dessins annexés, réalisés à titre d'exemples indicatifs et non limitatifs et sur lesquels :
- la figure 1a est une vue schématique montrant les différents éléments constitutifs du champ de bordure chirurgicale conforme à l'invention,
- la figure 1b est une vue schématique montrant les différents éléments constitutifs de la figure 1 a aménagés de manière à former le champ de bordure chirurgicale conforme à l'invention,
- la figure 2 est une vue schématique montrant, de face, l'agencement des différents éléments constitutifs de la partie femelle du champ de bordure chirurgicale conforme à l'invention,
- la figure 3 est une vue en coupe selon A-A de la partie femelle du champ de bordure chirurgical représenté sur la figure 2,
- la figure 4 est une vue schématique montrant, en coupe, le dispositif de protection de l'invention mis en place contre les berges d'incision.

Le dispositif de protection objet de l'invention est destiné à être utilisé lors d'opérations chirurgicales dans le but de protéger les berges d'incision. Il est mis en place après l'opération d'incision.

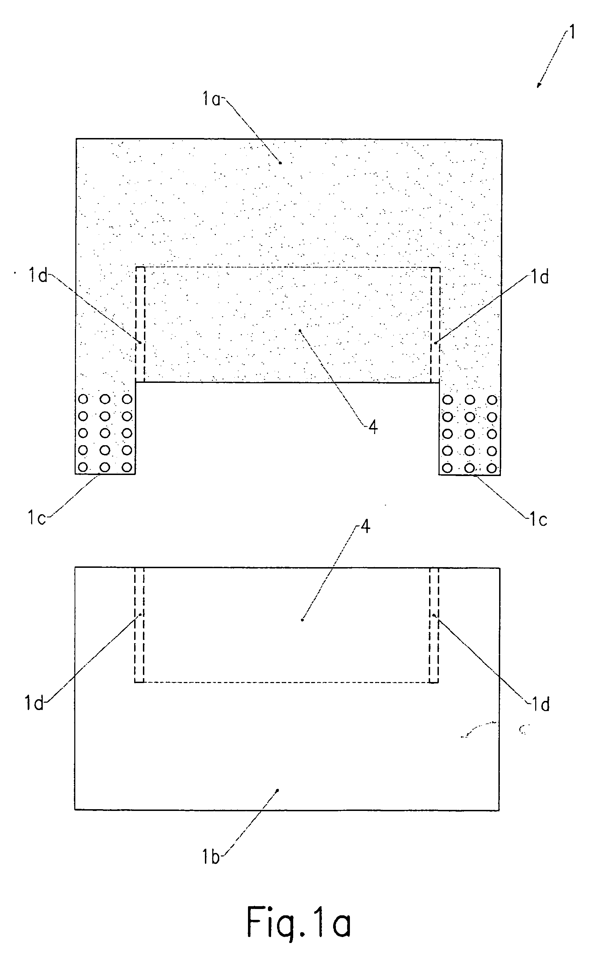

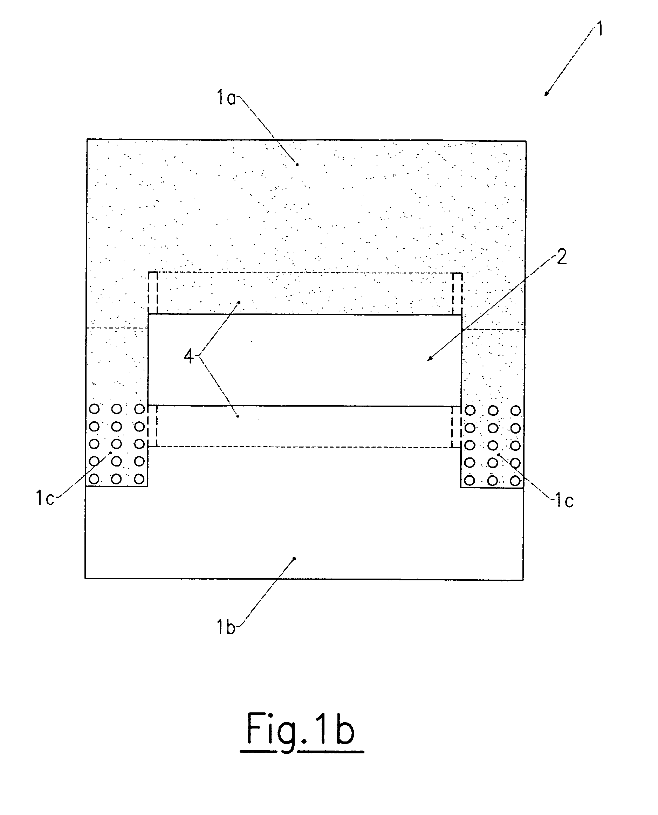

En se rapportant aux figures 1a et 1b, le dispositif de protection conforme à l'invention comporte un champ de bordure chirurgical 1 équipé d'une fenêtre d'incision 2, partielle ou totale, au travers de laquelle le chirurgien réalisera son intervention.

Le champ de bordure chirurgical 1 peut être réalisé en une seule pièce, mais est avantageusement formé d'une partie mâle 1a et d'une partie femelle 1b séparables (figure 1a), la fenêtre d'incision 2 étant aménagée au niveau de la jonction entre ladite partie mâle 1a et ladite partie femelle 1b (figure 1b). L'utilisation d'un champ chirurgical en deux parties séparables simplifie sa conception et sa mise en place par le chirurgien.

Selon une caractéristique avantageuse de l'invention permettant d'adapter la taille de la fenêtre d'incision 2 à la taille de l'incision à réaliser, la partie mâle 1a et la partie femelle 1b du champ de bordure chirurgical 1 sont maintenues en position par un dispositif de fixation réglable permettant d'ajuster la position de ladite partie mâle par rapport à ladite partie femelle et de faire varier la taille de ladite fenêtre d'incision.

En se référant aux figures 1a et 1b, le dispositif de fixation réglable est préférentiellement formé par des pattes de fixation 1c adhésives agencées sur la partie mâle 1a et aptes à adhérer sur la partie femelle 1b. Les zones adhésives peuvent éventuellement être protégées par un papier de protection amovible que l'opérateur enlèvera juste avant la mise en place. Tout autre dispositif de fixation réglable équivalent peut être utilisé par l'homme du métier, par exemple des bandes à boucles et à crochet (VELCRO®), ou des éléments mâles s'insérant dans des éléments femelles.

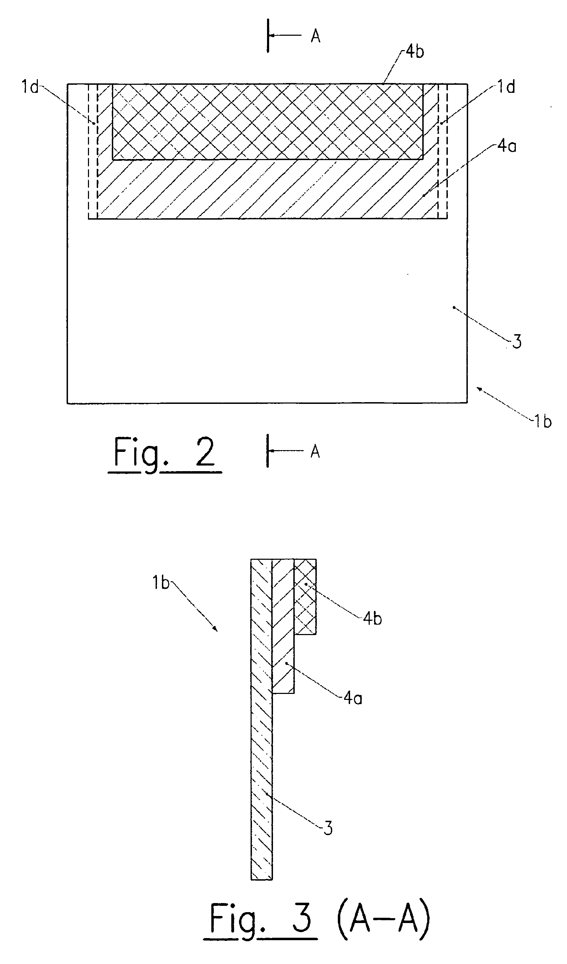

Conformément aux figures 1a, 1b et 2, la partie femelle 1 b et la mâle 1 a sont à bordures droites, avec des incisions 1d sur chaque coté aménagées de manière à ce que les bordures de la fenêtre d'incision 2 puissent être facilement introduites dans la berge chirurgicale tout en maintenant les pattes de fixation 1c à l'extérieur du corps du patient. Les incisions 1d sont avantageusement configurées de manière à ce qu'environ les 4/5^{ème} du champ puisse être introduit dans la berge chirurgicale. Selon une caractéristique avantageuse de l'invention, les incisions 1d sont prédécoupées de manière à ce que le praticien puisse introduire tout ou partie des bordures de la fenêtre d'incision 2 dans la berge chirurgicale, à sa convenance.

Le champ de bordure chirurgical 1 comporte une structure de base 3 réalisée dans un matériau stérile non tissé du type ouate de cellulose, mono-couche ou multi-couches. Tout autre matériau connu de l'homme du métier et convenant à la fabrication d'un champ de bordure chirurgical peut être employé. La structure de base 3 a une largeur comprise entre 20 cm et 40 cm, une longueur totale comprise entre 20 et 60 cm et une épaisseur variant de 1 mm à 2 mm. Toutefois, ces dimensions ne sont pas limitatives et peuvent être adaptées par l'homme du métier en fonction du type d'intervention chirurgicale réalisée.

Dans le but d'isoler efficacement le corps du patient de l'environnement extérieur et notamment de l'introduction d'un fluide contaminé, la face supérieure du champ de bordure chirurgical 1 est imperméabilisée. Cette imperméabilisation peut se faire au moyen d'un film plastique, d'un matériau imperméable aux liquides, d'un matériau hydrophobe ou tout autre matériau convenant à l'homme du métier.

Sur les figures 2 et 3, seule est représentée la partie femelle 1b du champ de bordure chirurgicale 1. Toutefois, les caractéristiques représentées s'appliquent également à la partie mâle 1a ou au champ de bordure 1 dans le cas où celui-ci est réalisé en une seule pièce.

Conformément à l'invention et en se rapportant aux figures 1a et 1b, la face interne des bordures de la fenêtre d'incision 2 est recouverte d'un moyen 4 pour protéger les berges d'incision.

Conformément à l'invention, ce moyen de protection 4 comporte une couche 4b imprégnée d'un produit hémostatique antiseptique et/ou bactéricide et/ou antibactérien et/ou antibiotique et/ou présentant un pH acide.

Selon une caractéristique avantageuse de l'invention représentée sur la figure 2 et permettant d'optimiser la protection des berges d'incision, le moyen de protection 4 comporte une première couche 4a d'un matériau destiné à absorber les secrétions d'incision et la seconde couche 4b imprégnée d'un produit hémostatique antiseptique et/ou bactéricide et/ou antibactérien et/ou antibiotique et/ou présentant un pH acide.

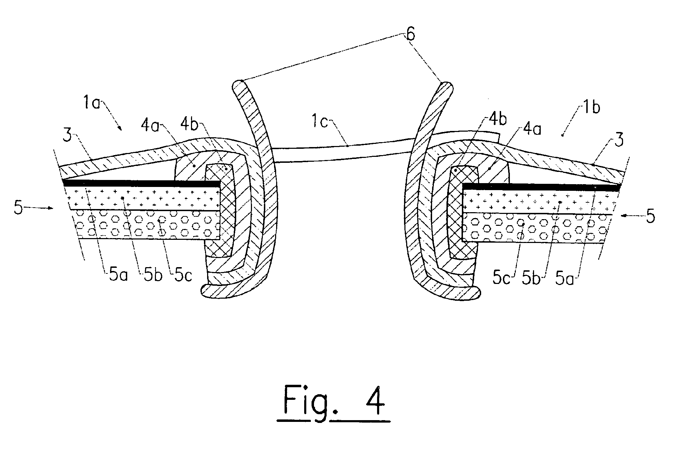

En se rapportant à la figure 4, la seconde couche 4b est aménagée de manière à ce qu'elle vienne au contact des berges d'incision 5 lors de la mise en position du champ de bordure chirurgical 1 autour de l'incision. Par « berges d'incision », on entend au sens de la présente invention le plan cutané 5a et/ou le plan sous-cutané 5b et/ou le plan osseux, musculaire ou aponévrotique 5c.

Selon une caractéristique avantageuse de l'invention représentée sur les figures, la première couche 4a et la seconde couche 4b sont disposées immédiatement adjacent aux bordures de la fenêtre d'incision 2 de façon à permettre au chirurgien de protéger rapidement les berges d'incision 5 par le simple rabattement desdites bordures dans le corps du patient.

Et dans le but de protéger parfaitement les berges d'incision 5, la seconde couche 4a est avantageusement aménagée de manière à ce qu'elle vienne en contact desdites berges et recouvre la partie supérieure et la partie inférieure de l'incision lors de la mise en place d'un écarteur 6. En effet, tout au long de l'intervention, ce dernier vient impacter et parfaitement maintenir en position le dispositif de protection objet de l'invention contre les berges 5, sans que les bordures de la fenêtre d'incision 2 viennent gêner le chirurgien.

La première couche 4a peut être de n'importe quel type conventionnel. Les matériaux absorbants couramment rencontrés sont par exemple la pâte de cellulose, des polymères hautement absorbants, des matériaux mousse absorbants, des fibres non tissées absorbantes. Il est possible de combiner ces différents matériaux de manière à avoir des propriétés différentes en ce qui concerne la capacité d'absorption de liquide, la capacité de répartition, et la capacité de stockage. Le matériau utilisé est de préférence, mais pas nécessairement, une compresse stérile épaisse absorbante de type HYDRE®.

La seconde couche 4b est préférentiellement réalisée par l'intermédiaire d'une compresse stérile de type HYDREX® imprégnée d'un produit hémostatique antiseptique et/ou bactéricide et/ou antibactérien et/ou antibiotique et/ou présentant un pH acide. Tout autre matériau ou revêtement équivalent apte à être imprégné de tels produits peut être utilisé par l'homme du métier. Cette imprégnation a pour but d'éviter le développement des infections, des abcès de parois ou encore des hématomes. Parmi les produits d'imprégnation utilisables, on peut citer à titre d'exemple non limitatif : les produits hémostatiques tels que SURGICEL® ou PANGEN®, ...), peroxyde d'oxygène les antiseptiques à base d'ions d'Argent, alginate de Calcium, Piliostigma Reticulatum, les agents bactériostatiques, les agents bactéricides, les molécules antibiotiques, les molécules à pH acide, etc. Ces différents produits d'imprégnation peuvent être utilisés seuls ou en combinaison.

La seconde couche 4b vient couvrir totalement ou partiellement la première couche 4b. En se rapportant aux figures 2 et 3, la première couche 4a est plus large que la seconde couche 4b afin que ladite première couche puisse absorber et stocker efficacement les saignements ou autres sécrétions corporelles. En pratique, la première couche 4a a une longueur sensiblement égale à la largeur du champ de bordure chirurgicale 1 (et de la partie mâle 1a et femelle 1 b), une largeur d'environ 10 cm et une épaisseur variant de 4 mm à 6 mm. La seconde couche 4b a également une longueur sensiblement égale à la largeur du champ de bordure chirurgicale 1 (et de la partie mâle 1a et femelle 1b), une largeur d'environ 5 cm et une épaisseur variant de 4 mm à 6 mm. Toutefois, ces dimensions ne sont pas limitatives et peuvent être adaptées par l'homme du métier en fonction le type d'intervention chirurgicale réalisée.

## Revendications

1. Dispositif de protection actif des berges d'incision (5) destiné à éviter le développement des infections, comportant un champ de bordure chirurgical (1) dans lequel est aménagée une fenêtre d'incision (2) totale ou partielle, **se caractérisant par le fait que** :
- la face interne des bordures de la fenêtre d'incision (2) est recouverte d'une première couche (4a) d'un matériau destiné à absorber les secrétions d'incision et d'une seconde couche (4b) imprégnée d'un produit hémostatique antiseptique ayant pour but d'éviter le développement des infections,
- la seconde couche (4b) vient couvrir partiellement la première couche (4a), ladite première couche étant plus large que ladite seconde couche,
- la seconde couche (4b) est aménagée de manière à ce qu'elle puisse venir au contact des berges d'incision (5) et recouvre la partie supérieure et la partie inférieure de l'incision lors de la mise en place d'un écarteur (6).

2. Dispositif selon la revendication 1, dans lequel le champ de bordure chirurgical (1) est formé d'une partie mâle (1a) et d'une partie femelle (1 b) séparables, la fenêtre d'incision (2) étant aménagée au niveau de la jonction entre les deux dites parties.

3. Dispositif selon la revendication 2, dans lequel la partie mâle (1a) et la partie femelle (1b) du champs de bordure chirurgicale (1) sont maintenues en position par un dispositif de fixation réglable permettant d'ajuster la position de ladite partie mâle par rapport à ladite partie femelle et de faire varier la taille de la fenêtre d'incision (2).

4. Dispositif selon la revendication 3, dans lequel le dispositif de fixation réglable est formé par des pattes de fixation (1c) adhésives agencées sur la partie mâle (1a) et aptes à adhérer sur la partie femelle (1 b).

5. Dispositif selon la revendication 4, dans lequel la partie femelle (1b) et la partie mâle (1a) sont à bordures droites, avec des incisions (1d) sur chaque côté aménagées de manière à ce que les bordures de la fenêtre d'incision (2) puissent être introduites dans la berge chirurgicale tout en maintenant les pattes de fixation (1c) à l'extérieur du corps du patient.

6. Dispositif selon la revendication 5, dans lequel les incision (1d) sont configurées de manière à ce qu'environ les 4/5ème du champ de bordure chirurgical (1) puisse être introduite dans la berge chirurgicale.

7. Dispositif selon l'une des revendications 5 ou 6, dans lequel les incision (1d) sont prédécoupées de manière à ce que tout ou partie des bordures de la fenêtre d'incision (2) puisse être introduite dans la berge chirurgicale.

8. Dispositif selon l'une des revendications précédentes, dans lequel la première couche (4a) et la seconde couche (4b) sont disposées immédiatement adjacent aux bordures de la fenêtre d'incision (2).

9. Dispositif selon l'une des revendications précédentes, dans lequel la couche (4b) imprégnée d'un produit hémostatique antiseptique et également imprégné d'un produit bactéricide et/ou antibactérien et/ou antibiotique et/ou présentant un pH acide.

10. Dispositif selon l'une des revendications précédentes, dans lequel la face supérieure du champ de bordure chirurgical (1) est imperméabilisée.

## Claims

1. Device for active protection of incision margins (5), intended to prevent development of infections and having a surgical drape (1) in which a total or partial incision window (2) is formed, **characterized in that**:
- the inner face of the borders of the incision window (2) is covered by a first layer (4a) of a material intended to absorb the incision secretions, and by a second layer (4b) impregnated with an antiseptic haemostatic product whose purpose is to prevent development of infections,
- the second layer (4b) partially covers the first layer (4a), said first layer being wider than said second layer,
- the second layer (4b) is formed in such a way that it can come into contact with the incision margins (5) and covers the upper part and the lower part of the incision upon placement of a retractor (6).

2. Device according to Claim 1, in which the surgical drape (1) is formed by a male part (1a) and a female part (1b) that are separable, the incision window (2) being formed at the junction between said two parts.

3. Device according to Claim 2, in which the male part (1a) and the female part (1b) of the surgical drape (1) are held in position by an adjustable fixing device with which it is possible to adjust the position of said male part relative to said female part and to vary the size of the incision window (2).

4. Device according to Claim 3, in which the adjustable fixing device is formed by adhesive fixing tabs (1c) that are arranged on the male part (1a) and are able to adhere to the female part (1b).

5. Device according to Claim 4, in which the female part (1b) and the male part (1a) have straight edges, with incisions (1d) on each side that are formed in such a way that the borders of the incision window (2) can be introduced into the surgical margin while maintaining the fixing tabs (1c) outside the body of the patient.

6. Device according to Claim 5, in which the incisions (1d) are configured in such a way that about 4/5th of the surgical drape (1) can be introduced into the surgical margin.

7. Device according to one of Claims 5 and 6, in which the incisions (1d) are pre-cut in such a way that all or part of the borders of the incision window (2) can be introduced into the surgical margin.

8. Device according to one of the preceding claims, in which the first layer (4a) and the second layer (4b) are arranged immediately adjacent to the borders of the incision window (2).

9. Device according to one of the preceding claims, in which the layer (4b) impregnated with an antiseptic haemostatic product is also impregnated with a product having a bactericidal and/or antibacterial and/or antibiotic action and/or having an acid pH.

10. Device according to one of the preceding claims, in which the upper face of the surgical drape (1) is rendered impermeable.

## Patentansprüche

1. Vorrichtung zum aktiven Schutz von Inzisionsrändern (5), die dazu bestimmt ist, die Entwicklung von Infektionen zu vermeiden, mit einem chirurgischen Abdecktuch (1), in dem ein ganzes oder ein partielles Inzisionsfenster (2) vorgesehen ist, **dadurch gekennzeichnet, dass**
- die Innenfläche der Ränder des Inzisionsfensters (2) mit einer ersten Schicht (4a) aus einem Material abgedeckt ist, das dazu bestimmt ist, die Inzisionssekrete zu absorbieren, und mit einer zweiten Schicht (4b), die mit einem blutstillenden antiseptischen Produkt imprägniert ist, um die Entwicklung von Infektionen zu verhindern,
- die zweite Schicht (4b) die erste Schicht (4a) teilweise abdeckt, wobei die erste Schicht größer als die zweite Schicht ist,
- die zweite Schicht (4b) so angeordnet ist, dass sie mit den Inzisionsrändern (5) in Kontakt kommen kann und bei Einsatz eines Spreizers (6) den oberen Teil und den unteren Teil der Inzision abdeckt.

2. Vorrichtung nach Anspruch 1, wobei das chirurgische Abdecktuch (1) aus einem männlichen Teil (1a) und einem weiblichen Teil (1b) gebildet ist, die trennbar sind, wobei das Inzisionsfenster (2) an der Stoßstelle zwischen den beiden Teilen angeordnet ist.

3. Vorrichtung nach Anspruch 2, wobei der männliche Teil (1a) und der weibliche Teil (1b) des chirurgischen Abdecktuchs (1) durch eine verstellbare Befestigungsvorrichtung in Position gehalten werden, mittels derer die Position des männlichen Teils bezüglich des weiblichen Teils verstellt und die Größe des Inzisionsfensters (2) verändert werden kann.

4. Vorrichtung nach Anspruch 3, wobei die verstellbare Befestigungsvorrichtung aus Befestigungsklebelaschen (1c) gebildet ist, die am männlichen Teil (1a) angeordnet und geeignet sind, am weiblichen Teil (1b) zu haften.

5. Vorrichtung nach Anspruch 4, wobei der weibliche Teil (1b) und der männliche Teil (1a) gerade Ränder haben, wobei Inzisionen (1d) so an jeder Seite angeordnet sind, dass die Ränder des Inzisionsfensters (2) in den chirurgischen Wundrand eingeführt werden können, während die Befestigungslaschen (1c) an der Außenseite des Körpers des Patienten gehalten werden.

6. Vorrichtung nach Anspruch 5, wobei die Inzisionen (1d) so konfiguriert sind, dass ungefähr 4/5 des chirurgischen Abdecktuchs (1) in den chirurgischen Wundrand eingeführt werden kann.

7. Vorrichtung nach Anspruch 5 oder 6, wobei die Inzisionen (1d) so vorgeschnitten sind, dass die Ränder des Inzisionsfensters (2) ganz oder teilweise in den chirurgischen Wundrand eingeführt werden können.

8. Vorrichtung nach einem der vorhergehenden Ansprüche, wobei die erste Schicht (4a) und die zweite Schicht (4b) unmittelbar neben den Rändern des Inzisionsfensters (2) angeordnet sind.

9. Vorrichtung nach einem der vorhergehenden Ansprüche, bei der die Schicht (4b) mit einem blutstillenden antiseptischen Produkt imprägniert ist sowie auch mit einem bakteriziden und/oder antibakteriellen und/oder antibiotischen Produkt und/oder einem Produkt mit saurem pH-Wert imprägniert ist.

10. Vorrichtung nach einem der vorhergehenden Ansprüche, wobei die obere Fläche des chirurgischen Abdecktuchs (1) flüssigkeitsdicht gemacht ist.
